# EUROPÄISCHE PATENTANMELDUNG

(11) **EP 2 700 386 A1**
(43) Veröffentlichungstag der Anmeldung: **26.02.2014**
(21) Anmeldenummer: 13003976.1
(22) Anmeldetag: 09.08.2013
(51) Int. Cl.: A61F 5/44, B65D 33/16, A61M 25/00, B65D 81/32

(54) **Benetzungsvorrichtung mit lösbar verriegelter Klammer**

(30) Priorität: 20.08.2012 DE 102012016507
(71) Anmelder: Medical Service GmbH, 75378 Bad Liebenzell (DE)
(72) Erfinder: Hiesch, Bernhard, 75328 Schömberg (DE)
(74) Vertreter: Grünecker, Kinkeldey, Stockmair & Schwanhäusser

(57) **Zusammenfassung**

Die vorliegende Anmeldung betrifft eine Benetzungsvorrichtung (1) für in den menschlichen Körper einführbare medizinische Geräte mit einem Folienbeutel (2), der eine erste Kammer (3) zur Aufnahme des medizinischen Geräts und eine zweite Kammer (4) mit einem darin angeordneten Benetzungsmedium (6) umfasst, wobei die erste Kammer und die zweite Kammer durch eine an den Außenseiten des Folienbeutels angebrachte Klammer (5) voneinander getrennt sind. Es ist die Aufgabe der vorliegenden Anmeldung, eine Benetzungsvorrichtung bereitzustellen, die die aus dem Stand der Technik bekannten Geräte verbessert, einen flüssigkeitsdichten Verschluss ermöglicht und ein einfaches Handhaben gewährleistet. Hierzu ist vorgesehen, dass die Klammer zwei ineinandergreifende Leisten (10, 11) umfasst, die den Folienbeutel zwischen sich einschließen, wobei eine der Leisten eine Hinterschneidung (14) ausbildende Nut (12) aufweist und die zweite Leiste eine zu der Nut komplementäre Rippe (14) ausbildet.

## Beschreibung

Die vorliegende Erfindung betrifft eine Benetzungsvorrichtung für in den menschlichen Körper einführbare medizinische Geräte mit einem Folienbeutel, der eine erste Kammer zur Aufnahme des medizinischen Geräts und eine zweite Kammer mit einem darin angeordneten Benetzungsmedium umfasst, wobei die erste Kammer und die zweite Kammer durch eine an den Außenseiten des Folienbeutels angebrachte Klammer voneinander getrennt sind.

Es ist bekannt, die Außenfläche von medizinischen Instrumenten mit einem Benetzungsmedium zu benetzen, um die Gleitreibung beim Einführen des medizinischen Geräts in Hohlorgane des menschlichen Körpers zu reduzieren. Die medizinischen Geräte können dann möglichst schmerzfrei und ohne weitere Irritationen eingeführt werden.

Ein typischer Einsatzbereich für solche Benetzungsvorrichtungen ist beispielsweise der Einmalkatheterismus. Ist die natürliche Blasenfunktion nicht mehr gegeben, so muss die Harnblase wiederholt durch dünne Einmalkatheter entleert werden. Dieser Vorgang wird auch intermittierender Katheterismus genannt. Dabei wird ein dünner Einmalkatheter durch die Harnröhre in die Blase eingeführt und der Urin über den Katheter abgelassen. Durch das Aufbringen eines Gleitmittels auf den Katheter werden Irritationen der Harnröhre beim Einbringen erheblich verringert. So ist es beispielsweise bekannt, Katheter mit Gleitgel zu beschichten. Zudem gibt es Katheter, die eine hydrophile Beschichtung aufweisen, welche vor dem Einbringen des Katheters in die Harnröhre aktiviert werden muss. Beispielsweise durch die Benetzung mit Wasser. Um einen einfachen Katheterismus auch unterwegs zu ermöglichen, sind mittlerweile gebrauchsfertige Kathetersets bekannt, die einen Einmalkatheter umfassen, der zusammen mit dem Benetzungsmedium in einer Verpackung angeordnet ist.

So zeigt die EP 1 982 741 A2 bereits ein Katheterset, in dem ein Katheter und das Benetzungsmedium für den Katheter in einer gemeinsamen Verpackung angeordnet sind. Die Verpackung umfasst zwei Kammern, die durch eine Schweißnaht voneinander getrennt sind. In einer der Kammern ist der Katheter angeordnet, in der anderen Kammer das Benetzungsmedium. Der Benutzer drückt vor Gebrauch des Katheters auf die Kammer mit dem Benetzungsmedium, die Schweißnaht gibt nach und der Katheter wird benetzt.

Ein weiteres gebrauchsfertiges Blasenkatheterset ist beispielsweise in der EP 0 923 398 B1 beschrieben. Das Blasenkatheterset umfasst einen zumindest teilweise hydrophil beschichteten Blasenkatheter, und eine Katheterpackung, die einen Hohlraum zur Aufnahme des Katheters ausbildet. Die Katheterverpackung umfasst ferner ein Kompartiment, in dem das Benetzungsmedium angeordnet ist. Das Kompartiment ist von dem Hohlraum, in dem der Katheter angeordnet ist, abgetrennt, wobei die Abtrennung derart ausgebildet ist, dass sie zum Benetzen des Katheters aufgebrochen werden kann. So ist beispielsweise gezeigt, dass das Kompartiment durch die Verpackung selbst ausgebildet ist und mittels einer Klammer, die außen an der Verpackung angebracht ist und die Wände der Katheterverpackung zusammendrückt, gegenüber dem Hohlraum, in dem der Katheter angeordnet ist, verschlossen ist.

Nachteilig an diesem Katheterset ist, dass die Kammer mit dem Benetzungsmedium von einem Benutzer, der üblicherweise in seiner Beweglichkeit eingeschränkt ist, nur schwer geöffnet werden kann. Zudem muss das Benetzungsmedium sicher getrennt von dem Katheter angeordnet werden.

Es ist daher die Aufgabe der vorliegenden Erfindung, eine Benetzungsvorrichtung bereitzustellen, die die aus dem Stand der Technik bekannten Vorrichtungen weiter verbessert. Insbesondere soll eine hohe Benutzerfreundlichkeit bei einem gleichzeitig sicher flüssigkeitsdichten Verschluss erzielt werden.

Hierzu ist erfindungsgemäß vorgesehen, dass die Klammer zwei ineinandergreifende Leisten umfasst, die den Folienbeutel zwischen sich einschließen, wobei eine der Leisten eine eine Hinterschneidung ausbildende Nut aufweist und die zweite Leiste eine zu der Nut komplementäre Rippe ausbildet, wobei die Klammer derart ausgebildet ist, dass sie sich durch eine quer zu ihrer Längserstreckung ausgeübte Krafteinwirkung öffnet.

Im geschlossenen Zustand der Klammer sind die Wände des Folienbeutels zwischen den beiden Leisten der Klammer eingeklemmt. Die zweite Leiste der Klammer mit der Rippe ist durch die Hinterschneidung sicher in der Nut der ersten Leiste aufgenommen. Ein versehentliches Öffnen wird vermieden. Zudem wird ein sehr dichter Verschluss erzielt. Es wird eine sehr einfache Ausgestaltung des Verschlusses ermöglicht. Die Klammer kann beispielsweise dadurch geöffnet werden, dass der Benutzer den Folienbeutel an beiden Seiten neben der Klammer oder an den Enden des Folienbeutels greift und an dem Folienbeutel zieht. Eine andere Möglichkeit zum Öffnen der Klammer besteht darin, dass der Benutzer die zweite Kammer mit dem darin angeordneten Benetzungsmedium zusammendrückt, dadurch komprimiert und einen Druck auf die Leisten der Klammern ausübt. Somit kann die zweite Kammer, die das Benetzungsmedium enthält, auch von einem in seiner Beweglichkeit eingeschränkten Benutzer sehr einfach geöffnet werden und das Benetzungsmedium mit dem zu benetzenden medizinischen Gerät in Kontakt gebracht werden.

Zwar sind aus der DE 76 06 374 U und der DE 72 02 949 U bereits Klammerverschlüsse für Folien bekannt, die ineinander greifende Leisten umfassen. Allerdings bezieht sich die DE 76 06 374 U auf eine Verpackung für einen Zweikomponentenkleber im Baugewerbe, bei dem die Klammer durch Auseinanderschieben der Leisten geöffnet wird. Die DE 72 02 949 U bezieht sich auf die Verbindung von zwei Folienbahnen, z.B. im landwirtschaftlichen Bereich. Zum öffnen und Schließen der Leisten ist ein Schieber vorgesehen. Die Leisten sind so ausgestaltet, dass sie bei Querzugbelastung geschlossen bleiben.

Vorteilhafterweise kann ferner vorgesehen sein, dass die Klammer derart ausgebildet ist, dass sie auslöst, wenn ein auf die zweite Kammer mit dem darin angeordneten Benetzungsmedium ausgeübter Druck 0,15 N/mm² (1,5 bar) übersteigt. Durch eine derart ausgebildete Klammer wird ein versehentliches Öffnen der zweiten Kammer mit dem Benetzungsmedium vermieden, dennoch ist ein einfaches Öffnen dieser zweiten Kammer durch den in seiner Beweglichkeit eingeschränkten Benutzer gegeben.

Gemäß einer weiteren Ausgestaltung kann ferner vorgesehen sein, dass der Folienbeutel eine Druckbeständigkeit aufweist, die mindestens das Zweifache der Auslösekraft beträgt. Diese Druckbeständigkeit gilt dabei sowohl für eventuell vorhandene Verschlussnähte des Folienbeutels als auch für das Material des Folienbeutels selbst. Es wird dadurch sichergestellt, dass beim Auslösen der Benetzungsvorrichtung das Benetzungsmedium in dem Folienbeutel verbleibt und nicht nach außen austritt.

Gemäß noch einer weiteren vorteilhaften Ausführungsform kann vorgesehen sein, dass die Nut der ersten Leiste und die Rippe der zweiten Leiste abgerundete Konturen aufweisen. Durch diese abgerundeten Konturen wird die Gefahr verringert, dass der Folienbeutel beim Öffnen oder Schließen der Klammer eingerissen wird und somit das Benetzungsmedium ausläuft.

Um eine einfache Ausgestaltung und Fertigung zu ermöglichen, kann vorgesehen sein, dass die erste Leiste die Form eines Rohrs mit einem in Längsrichtung verlaufenden Schlitz hat und die zweite Leiste als in das Rohr eingreifender Stab ausgebildet ist.

Ferner kann auch vorgesehen sein, dass die Klammer an dem Folienbeutel befestigt ist. Auch nach dem Öffnen der Klammer bleibt diese daher an dem Folienbeutel hängen, es ist eine einfache Handhabung und Entsorgung der Benetzungsvorrichtung nach jeder Benutzung möglich.

In noch einer weiteren Ausgestaltung kann vorgesehen sein, dass die beiden Leisten der Klammer an einem Ende gelenkig miteinander verbunden sind. Auch nach dem Öffnen bleiben die beiden Leisten daher miteinander verbunden. Dadurch wird eine Wiederverschließbarkeit gewährleistet. Zudem können beide Leisten nach dem Öffnen mit dem Folienbeutel verbunden bleiben, so dass die Handhabung und Entsorgung erleichtert wird.

In diesem Fall kann die Befestigung der Klammer an dem Folienbeutel dadurch realisiert werden, dass lediglich eine der Leisten der Klammer dauerhaft mit dem Folienbeutel verbunden ist.

Noch eine weitere Ausführungsform kann vorsehen, dass die Klammer einen Originalitätsverschluss aufweist, der beim Öffnen der Klammer zerstört wird. Durch diesen Originalitätsverschluss kann vor Benutzung der Benetzungsvorrichtung überprüft werden, ob sich die Benetzungsvorrichtung noch im Originalzustand befindet und die nötige Sterilität gegeben ist.

Im Folgenden wird die Erfindung anhand von Zeichnungen näher erläutert. Es zeigen:
Fig. 1 Draufsicht auf eine erfindungsgemäße Benetzungsvorrichtung und
Fig. 2 Schnitt durch einen Teilbereich der Benetzungsvorrichtung aus Fig. 1 entlang der Linie 11-11 in vergrößerter Darstellung.

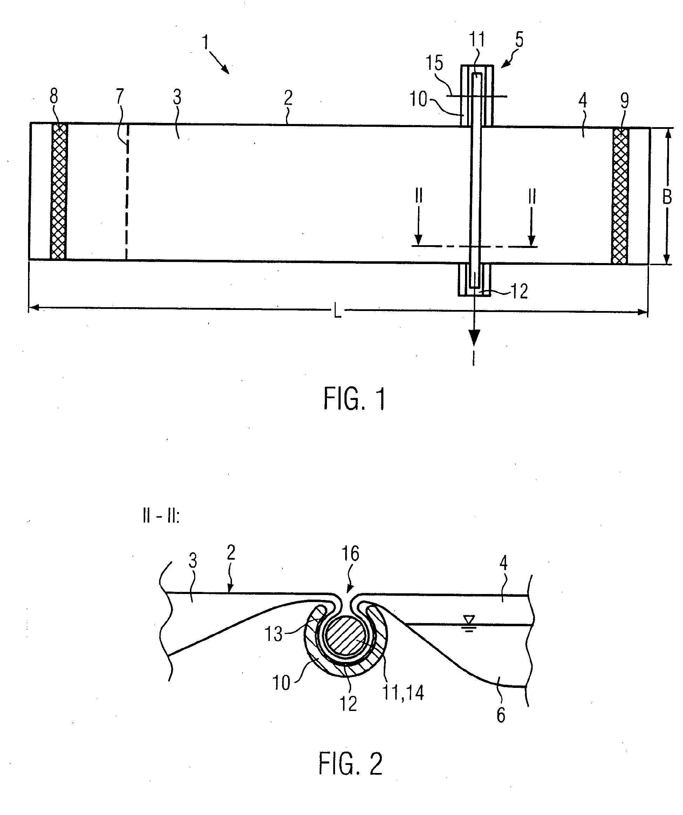

Fig. 1 zeigt eine Draufsicht auf eine erfindungsgemäße Benetzungsvorrichtung 1. Die Benetzungsvorrichtung 1 umfasst einen Folienbeutel 2, der eine erste Kammer 3 und eine zweite Kammer 4 ausbildet. Die erste Kammer 3 und die zweite Kammer 4 sind durch eine Klammer 5 voneinander getrennt. In der ersten Kammer 3 ist das medizinische Gerät, beispielsweise ein Einmalkatheter angeordnet. In der zweiten Kammer 4 befindet sich das Benetzungsmedium 6. Bei dem Benetzungsmedium 6 kann es sich, je nach Ausgestaltung des Katheters, um ein Gleitgel oder auch um eine Kochsalzlösung oder steriles Wasser handeln. Im Bereich der ersten Kammer 3 weist der Folienbeutel 2 eine Sollbruchstelle 7 auf. An dieser Sollbruchstelle 7 kann der Folienbeutel 2 einfach geöffnet und das medizinische Gerät (nicht dargestellt) entnommen werden. Die Sollbruchstelle 7 muss derart ausgebildet sein, dass sie flüssigkeitsdicht ist.

Im dargestellten Fall ist der Folienbeutel 2 als schlauchförmiger Kunststoffbeutel ausgebildet, dessen Längserstreckung L deutlich größer ist als seine Breite B. In Längsrichtung L ist der Folienbeutel 2 an beiden Enden mit je einer Siegelnaht 8, 9 verschlossen. Sowohl die Siegelnaht 8 als auch die Siegelnaht 9 sind derart ausgebildet, dass sie einen flüssigkeitsdichten Verschluss des Folienbeutels 2 garantieren. Es wäre aber auch eine andere Ausgestaltung des Folienbeutels denkbar. Möglich ist beispielsweise, dass der Folienbeutel aus zwei Lagen aus Kunststofffolie besteht, die um den gesamten Umfang mit einer flüssigkeitsdichten Siegelnaht verschweißt sind.

Die Klammer 5 wird durch zwei Leisten 10, 11 ausgebildet. Die erste Leiste 10 weist eine Nut 12 auf, die eine Hinterschneidung 13 ausbildet. Die zweite Leiste 11 bildet eine Rippe 14 aus, wobei die Form der Rippe 14 in etwa der Form der Nut 12 entspricht. Die Rippe 14 ist also komplementär zur Nut 12 ausgebildet. An einem Ende sind die erste Leiste 10 und die zweite Leiste 11 durch ein Gelenk 15 miteinander verbunden. Zumindest eine der beiden Leisten 10, 11 ist dauerhaft mit dem Folienbeutel 2 verbunden, beispielsweise durch Verkleben. Es können aber auch beide Leisten mit dem Folienbeutel verbunden sein.

In Fig. 2 ist ein Schnitt durch den Folienbeutel 2 mit der darin angebrachten Klammer 5 entlang der Linie 11-11 aus Fig. 1 in Vergrößerung dargestellt. Die erste Leiste 10 hat die Form eines kreisförmigen Rohrs und ist in Längsrichtung der Klammer 5 mit einem Schlitz 16 versehen. Die zweite Leiste 11 ist als Stab mit einem kreisförmigen Querschnitt ausgebildet und im Inneren der ersten Leiste 10 angeordnet. Durch den Schlitz 16 wird die Hinterschneidung 13 ausgebildet, die die zweite Leiste 11 in der ersten Leiste 10 fixiert. Im dargestellten Beispiel ist die erste Leiste 10 außen an der Unterseite des Folienbeutels 2 angebracht. Die zweite Leiste 11 ist außen an der gegenüberliegenden Seite des Folienbeutels 2 angebracht, also an der Oberseite des Folienbeutels 2. Der Folienbeutel 2, bzw. die Wände des Folienbeutels 2, befindet sich zwischen der ersten Leiste 10 und der zweiten Leiste 11. Im geschlossenen Zustand der Klammer 5, wenn die zweite Leiste 11 in die erste Leiste 10 eingeklickt ist und durch die Hinterschneidung 14 dort fixiert ist, werden die Seitenwände des Folienbeutels 2 zwischen der ersten Leiste 10 und der zweiten Leiste 11 eingeklemmt. Dadurch wird ein flüssigkeitsdichter Verschluss ausgebildet. Wie in Fig. 2 dargestellt, verläuft der Folienbeutel 2 ausgehend von der ersten Kammer 3 in Richtung der Klemme 5, wird dort stark geknickt und zwischen der ersten Leiste 10 und der zweiten Leiste 11 eingeklemmt. Der Schlauchbeutel 2 folgt den Konturen der ersten Leiste 10 und der zweiten Leiste 11 und wird in der Klemme 5 in etwa kreisförmig geführt. In Richtung der zweiten Klammer 4 erfährt der Folienbeutel 2 bzw. die Wände des Folienbeutels 2 wieder einen starken Knick. Auch hierdurch wird die Flüssigkeitsdichtheit des Verschlusses garantiert. Die Konturen der ersten Leiste 10 und der zweiten Leiste 11 sind abgerundet, so dass Verletzungen des Folienbeutels 2 vermieden werden.

Die Klammer ist derart ausgebildet, dass sie sich durch eine Krafteinwirkung quer zu ihrer Längserstreckung I öffnet. Bei dieser Krafteinwirkung kann es sich um eine Zugkraft an beiden Seiten des Folienbeutels 2 links und rechts von der Klammer 5 handeln. Die Krafteinwirkung kann aber auch dadurch ausgeübt werden, dass die zweite Kammer 5, in der das Benetzungsmedium 6 angeordnet ist, komprimiert wird. Die Klammer ist derart ausgebildet, dass sie dann auslöst, wenn ein auf die zweite Kammer 4 mit dem Benetzungsmedium 6 ausgeübter Druck einen Wert von 0,15 N/mm² übersteigt. Durch die daraus realisierende Zug- bzw. Druckkraft werden die Wände des Folienbeutels 2 nach außen gezogen und dadurch die zweite Leiste 11 in Richtung des Schlitzes 16 bewegt. Um das gewünschte Auslösen der Klammer 15 zu ermöglichen, sollte die Breite des Schlitzes 16 mindestens ¾ des Durchmessers der Leiste 11 entsprechen.

Im Folgenden wird die Benutzung der Benetzungsvorrichtung 1 beschrieben. Zur Benetzung des in der Benetzungsvorrichtung 1 angeordneten medizinischen Geräts kann der Benutzer den Folienbeutel 2 an beiden Enden greifen und die Enden auseinander ziehen. Der zwischen den Leisten 10, 11 angeordnete Folienbeutel 2 bewegt dann die zweite Leiste 11 nach außen, aus der ersten Leiste 10 hinaus. Dieser Vorgang wird fortgeführt, bis die Klammer 5 komplett geöffnet ist. Da die Leisten 10, 11 durch das Gelenk 15 miteinander verbunden sind, bleiben sie auch im geöffneten Zustand aneinander hängen. Zudem ist mindestens eine der Leisten 10, 11 dauerhaft mit dem Folienbeutel 2 verbunden, so dass die gesamte Klammer 5 auch im geöffneten Zustand noch an dem Folienbeutel 2 anhängt. Ist die Klammer 5 nun vollständig geöffnet, so kann das Benetzungsmedium 2 aus der zweiten Kammer 4 in die erste Kammer 3 strömen und das dort angeordnete medizinische Gerät, beispielsweise einen Einmalkatheter, benetzen.

Als Alternative kann auch vorgesehen werden, dass der Benutzer zum Öffnen der Klammer 5 die zweite Kammer 4 des Folienbeutels 2, in der das Benetzungsmedium 6 angeordnet ist, mit einer Hand komprimiert. Auch dadurch wird der zwischen den beiden Leisten 10, 11 angeordnete Folienbeutel 12 nach außen gezogen und hebelt die zweite Leiste 11 aus der ersten Leiste 10 heraus, so dass die Klammer 5 geöffnet wird. Das Benetzungsmedium 6 strömt dann in die erste Kammer 3 ein und benetzt das dort angeordnete medizinische Gerät. Nach der Benetzung bzw. Aktivierung des medizinischen Geräts kann der Folienbeutel 2 an der Sollbruchstelle 7 geöffnet und das medizinische Gerät entnommen werden.

## Patentansprüche

1. Benetzungsvorrichtung (1) für in den menschlichen Körper einführbare medizinische Geräte mit einem Folienbeutel (2), der eine erste Kammer (3) zur Aufnahme des medizinischen Geräts und eine zweite Kammer (4) mit einem darin angeordneten Benetzungsmedium (6) umfasst, wobei die erste Kammer (3) und die zweite Kammer (4) durch eine an den Außenseiten des Folienbeutels (2) angebrachte Klammer (5) voneinander getrennt sind, **dadurch gekennzeichnet, dass** die Klammer (5) zwei ineinandergreifende Leisten (10, 11) umfasst, die den Folienbeutel (2) zwischen sich einschließen, wobei eine der Leisten (10) eine eine Hinterschneidung (13) ausbildende Nut (12) aufweist und die zweite Leiste (11) eine zu der Nut (12) komplementäre Rippe (14) ausbildet, und wobei die Klammer (5) derart ausgebildet ist, dass sie sich durch eine quer zu ihrer Längserstreckung (1) ausgeübte Krafteinwirkung öffnet.

2. Benetzungsvorrichtung nach Anspruch 1, **dadurch gekennzeichnet, dass** die Klammer (5) derart ausgebildet ist, dass sie auslöst, wenn ein auf die zweite Kammer (4) mit dem Benetzungsmedium (6) ausgeübter Druck 0,15 N/mm² übersteigt.

3. Benetzungsvorrichtung nach Anspruch 1 oder 2, **dadurch gekennzeichnet, dass** der Folienbeutel eine Druckbeständigkeit aufweist, die mindestens das zweifache der Auslösekraft beträgt.

4. Benetzungsvorrichtung nach einem der Ansprüche 1 bis 3, **dadurch gekennzeichnet, dass** die Nut (12) der ersten Leiste (10) und die Rippe (14) der zweiten Leiste (11) abgerundet Konturen aufweisen.

5. Benetzungsvorrichtung nach einem der Ansprüche 1 bis 4, **dadurch gekennzeichnet, dass** die erste Leiste (10) die Form eines Rohres mit einem in Längsrichtung verlaufenden Schlitz (16) hat und die zweite Leiste (11) als in das Rohr eingreifender Stab ausgebildet ist.

6. Benetzungsvorrichtung nach einem der Ansprüche 1 bis 5, **dadurch gekennzeichnet, dass** die Klammer (5) an dem Folienbeutel (2) befestigt ist.

7. Benetzungsvorrichtung nach einem der Ansprüche 1 bis 6, **dadurch gekennzeichnet, dass** die beiden Leisten (10, 11) der Klammer (5) an einem Ende gelenkig miteinander verbunden sind.

8. Benetzungsvorrichtung nach Anspruch 6 oder 7, **dadurch gekennzeichnet, dass** eine der Leisten (10, 11) der Klammer (5) dauerhaft mit dem Folienbeutel (2) verbunden ist.

9. Benetzungsvorrichtung nach einem der Ansprüche 1 bis 8, **dadurch gekennzeichnet, dass** die Klammer (5) einen Originalitätsverschluss aufweist, der beim Öffnen der Klammer (5) zerstört wird.
